(19) Europäisches Patentamt
European Patent Office
Office européen des brevets

(11) Publication number: **0 151 646**
**B1**

(12) # EUROPEAN PATENT SPECIFICATION

(45) Date of publication of patent specification: **07.10.87**

(51) Int. Cl.$^4$: **D 21 H 3/08**, D 21 H 3/60

(21) Application number: **84902826.1**

(22) Date of filing: **23.07.84**

(88) International application number:
**PCT/JP84/00373**

(87) International publication number:
**WO 85/00627 14.02.85 Gazette 85/04**

(54) **SIZING AGENT COMPOSITION AND METHOD OF USING THE SAME.**

(30) Priority: **22.07.83 JP 134051/83**

(43) Date of publication of application:
**21.08.85 Bulletin 85/34**

(45) Publication of the grant of the patent:
**07.10.87 Bulletin 87/41**

(84) Designated Contracting States:
**DE FR GB NL SE**

(56) References cited:
**GB-A-2 006 744**
**JP-A-50 160 505**
**JP-A-58 045 731**
**JP-A-58 087 398**

**Nishi Ichiro and two others (authors) "Kaimen Kasseizai Binran" 5 July 1960, Sangyo Tosho Kabushiki Kaisha, p. 20-21**

(73) Proprietor: **SEIKO KAGAKU KOGYO CO., LTD.**
**1-1, Tateishi 1-chome**
**Akashi-shi Hyogo 673 (JP)**
(73) Proprietor: **HOKUETSU PAPER MILLS, LTD.**
**5-1, Nishizaoh 3-chome**
**Nagaoka-shi Niigata 940 (JP)**
(73) Proprietor: **NIPPON OIL CO. LTD.**
**3-12, 1-chome Nishi-Shinbashi**
**Minato-ku Tokyo 105 (JP)**

(72) Inventor: **YOSHIOKA, Shigehiko**
**93-1, Morita Okubo-cho**
**Akashi-shi Hyogo 674 (JP)**
Inventor: **YOSHIDA, Tsuneo**
**2-19, Zaoh 2-chome**
**Nagaoka-shi Niigata 940 (JP)**
Inventor: **SATO, Hisatake**
**22-12, Nakatehara 1-chome Kohoku-ku**
**Yokohama-shi Kanagawa 222 (JP)**
Inventor: **YAMADA, Hideto**
**5-16-404, Oji 1-chome**
**Akashi-shi Hyogo 673 (JP)**
Inventor: **ADACHI, Yoshio**
**13-10, Kubo-cho**
**Nishinomiya-shi Hyogo 662 (JP)**

(74) Representative: **Heath, Derek James et al**
**Bromhead & Co. 30 Cursitor Street Chancery Lane**
**London EC4A 1LT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 151 646 B1**

Courier Press, Leamington Spa, England.

**Description**

The present invention relates to a sizing composition comprising an alkyl- or alkenyl substituted succinic anhydride, or a mixture of these succinic anhydrides, and polyoxyethylene alkyl ether phosphates or polyoxyethylene alkyl aryl ether phosphates or a mixture of these phosphates, and to a process for using the composition for the sizing agent by dispersing the composition in an aqueous solution of an aqueous soluble polymer compound, adding the thus obtained aqueous dispersant in a pulp slurry of paper making material, and making paper. Such a composition for sizing paper has excellent storage properties and, preferably, underwater self emulsion, while the process produces paper having an excellent sizing effect.

It is already known to produce a sizing agent, and particularly a paper making sizing agent, which comprises a mixture homogenized by mechanically agitating an aqueous solution of cationic starch and a substituted succinic anhydride in an aqueous dispersion to be mixed with a pulp slurry. It is also known to produce a dispersion of a mixture by premixing a substituted succinic anhydride and a nonionic surfactant of a certain type in water (JP—B—53.036.044), or an aqueous dispersion of a substituted succinic anhydride and underwater oil type surfactant dispersed in a substituted succinic anhydride having ultrafine particle diameters dispersed in aqueous solution of ampholitic acrylamide polymer (JP—A—58.045.731). Large scale apparatus is required in the case of producing the dispersion by using cationic starch of these aqueous dispersions, and it is difficult to pulverize sufficiently finely the dispersion particles in the obtained aqueous dispersion. Moreover, the sizing agent has a drawback, in that the sizing effect of the dispersion deteriorates, and the aqueous dispersions have inefficient storage stability. Thus, as time elapses, the underwater dispersant is erased and the sizing performance decreases considerably or does not perform as well as it might. Consequently, the mixture composition must be adjusted immediately prior to use as the sizing agent, which prevents a problem in the practical use of these compositions on an industrial scale.

In order to solve the above-described problems, it has been proposed to use an aqueous dispersion containing a surfactant having a substituted succinic anhydride and a half ester residue of the substituted succinic acid (JP—A—59 047 498). However, when a substituted alkenyl succinic anhydride of an addition reaction product of a straight chain internal olefin and maleic anhydride is used in an aqueous dispersion as the substituted succinic anhydride, sufficient underwater dispersing effect cannot be obtained unless the above-described surfactant is mixed in a large quantity, which disturbs the sizing effect. Therefore, an excellent sizing effect cannot be obtained.

Accordingly, an aim of the present invention is to provide a sizing composition which exhibits excellent storage stability and underwater self-emulsion and hence an excellent sizing effect. Another aim is to provide a process for effectively making paper having an excellent sizing effect by using the composition for the sizing agent, i.e., a process for using the composition for a sizing agent.

With these aims in mind, the invention is directed to a sizing composition comprising (a) 70—99.9 wt.% of an alkyl- or alkenyl succinic anhydride or a mixture of these succinic anhydrides, and (b) 0.1—30 wt.% of polyoxyethylene alkyl ether phosphates or polyoxyethylene alkyl aryl ether phosphates or a mixture of these phosphate esters.

As alkyl- or alkenyl substituted succinic anhydride in the composition for a sizing agent any succinic anhydride known as a sizing agent may be used, but preferably a substituted succinic anhydride having 8 or more carbons and, more preferably, having $C_{12}$—$C_{36}$ alkyl- or alkenyl groups. This substituted succinic anhydride can be readily produced generally by utilizing alpha-olefin, inner olefin or the addition reaction of an alpha-olefin, inner olefin or their mixture having carbon atoms of corresponding number or their mixture, and utilizing the addition reaction products of these mixtures with maleic anhydride. Particularly, when the substituted succinic anhydride is, for example, octadecene-9, tetradecene-7, hexadecene-7, eicosane-11 or their mixtures, straight chain inner olefin mixture in which the double bond produced by dehydrated reaction of a straight chain paraffin is substantially uniformly distributed at the positions except alpha-position, additive reaction product of inner olefin and maleic anhydride of straight chain inner olefin mixture having 70% or more of total amount of inner olefin disposed at 2-, 3- and 4-position of double bond produced by anisotropic reaction in the presence of a catalyst with straight alpha-olefin, i.e., substituted alkenyl succinic anhydride having succinic anhydride group in a substituted group, the sizing effect effected by the sizing composition of the present invention has been found to be excellent.

Polyoxyethylene alkyl ether phosphates or polyoxyethylene alkyl aryl ether phosphates are preferably compounds represented by the following general formula:

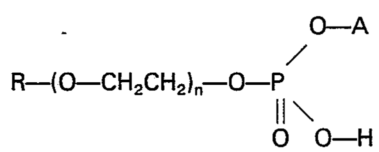

where R is an alkyl or alkyl aryl group having 8 or more carbon atoms, A represents H, or R'—(O—$CH_2CH_2$)$_m$— (R' is alkyl or alkyl aryl group, m is an integer of 1 or more), and n is an integer of 1 or more. Particularly when the R of the above general formula is an alkyl or alkyl aryl group having 10—20 carbon atoms and the value of n is 5 or more, the underwater dispersion particles of the produced

substituted succinic anhydride become extremely fine, and this enhances the sizing effect given by the composition of the present invention.

A mixture of (a) 70—90 wt.% of substituted succinic anhydride and (b) 10—30 wt.% of the phosphates produces a water dispersion containing substituted succinic anhydride of sufficiently fine particle size by merely mixing the mixture, thereby providing a so-called self-emulsion type paper making sizing agent. Further, a mixture of (a) 90—99.9 wt.% of substituted succinic anhydride and (b) 0.1—10 wt.% of the phosphates dispersed in aqueous solution of aqueous polymer compound produces an aqueous dispersion of substituted succinic anhydride of fine particle size which is sufficiently stable for mixing means providing extremely slow agitation. Therefore, when the mixture of (a) the substituted succinic anhydride and (b) the phosphates is used as a paper making sizing agent, it is preferable to use an aqueous dispersion utilizing an aqueous solution of aqueous soluble polymer compound so as to reduce the sizing effect disturbing action caused by the phosphates as much as possible.

The invention is also directed to a process for making paper which comprises dispersing a sizing composition having (a) 70—99.9 wt.% of alkyl- or alkenyl substituted succinic anhydride or a mixture of these succinic anhydrides and (b) 0.1—30 wt.% of polyoxyethylene alkyl ether phosphates or polyoxyethylene alkyl aryl ether phosphates or a mixture of these phosphates in an aqueous solution of an aqueous soluble polymer compound, adding the thus-obtained aqueous dispersion to a pulp slurry of paper making material, and making the paper in accordance with an ordinary paper-making method to provide sized paper.

In the aforementioned paper sizing process, the aqueous soluble polymer compound contained in the aqueous dispersion may employ not only cationic starch, ampholytic acrylic amide polymer heretofore known *per se*, but also other aqueous soluble starch derivatives, aqueous soluble cellulose derivatives, various acrylic amide polymers, polyvinylalcohol, polyethylene imine and its derivatives, aqueous soluble polyamide, aqueous soluble acrylic resin, aqueous soluble polyester, aqueous soluble maleic resin, and various aqueous soluble vinyl polymers. The aforementioned aqueous soluble polymer compounds contained in the aqueous soluble dispersion are preferably present in a range of 0.1—5 wt. parts, and preferably 0.5—2 wt. parts to 1 wt. parts, of substituted succinic anhydride in the aqueous soluble dispersion.

In the process for making paper using a sizing composition of the present invention, various additives such as a paper strength intensifier, a filtrate accelerator, a filler yield agent, a fixing agent or some other sizing agent may be arbitrarily introduced when the aqueous dispersion is added to the pulp slurry. As another process, the aqueous dispersion of the produced substituted succinic acid is coated on wet paper or semi-dried paper by suitable means known *per se* after paper making, thereby providing surface sizing.

The sizing composition of the present invention comprises a mixture of specific surfactant and substituted succinic anhydride. Since the surfactant has excellent solubility with respect to the substituted succinic anhydride, excellent storage stability is provided irrespective of the type of substituted succinic anhydride. Stable underwater self-emulsion is also provided, while an aqueous dispersion of substituted succinic anhydride having extremely fine particle size is provided in the presence of a small amount of surfactant. this leads to excellent results in the use of the composition in paper making.

Since a process for making paper of a method for using the sizing composition of the present invention adds the aqueous dispersion of substituted succinic anhydride having the above-described characteristics as a sizing agent in the paper making pulp slurry, it can eliminate the complexity of preparing the composition immediately before use without the use of large scale emulsifying apparatus to produce the aqueous dispersion. Therefore, it becomes possible to make paper having an excellent sizing effect on an industrial scale.

The present invention will now be described in further detail by means of preferred examples thereof as compared with reference examples. The properties of the aqueous dispersion of the sizing composition and the sizing effect of the paper will also be described.

Example 1

Comparison Example 1

Alkenyl substituted succinic anhydride and surfactant as listed in the columns of Table 1 are both agitated and mixed under slow heating conditions at ambient temperature or at 40°C or lower, and sizing compositions (a), (b), (c), (d) of the present invention and compositions (1), (2) for comparison are prepared.

The alkenyl substituted succinic anhydride A is maleic anhydride reacted with inner olefin having 15—18 carbon atoms, and the alkenyl substituted succinic anhydride B is maleic anhydride reacted with oligomer mainly containing pentamer of propylene.

TABLE 1

| Composition | Substituted alkenyl succinic anhydride | Surfactant |
|---|---|---|
| (a) | A 80 wt.% | Phosphates of polyoxyethylene nonyl phenol ether ester (mono-, di-mixture) (polyoxyethylene: n=9) 20 wt.% |
| (b) | B 85 wt.% | ditto 15 wt.% |
| (c) | A 85 wt.% | Phosphates of polyoxyethylene nonyl phenol ether ester (mono-, di-mixture) (polyoxyethylene: n=21.5) 15 wt.% |
| (d) | B 90 wt.% | ditto 10 wt.% |
| (1) | A 80 wt.% | Polyoxyethylene nonyl phenol ether (polyoxyethylene: n=15) 20 wt.% |
| (2) | B 85 wt.% | Sulfates of polyoxyethylene olenyl ether ester ammonium (polyoxyethylene: n=18) 15 wt.% |

Experiment 1

1 wt. parts of compositions (a) to (d) and (1), (2) provided in the example and comparison example are mixed with 99 wt. parts of water, agitated and dispersed to prepare aqueous dispersions of different types.

The prepared aqueous dispersions are added to 2 wt.% pulp slurry containing 20 wt.% of pulp (L-BKP, c.s.F. 400 cc) of heavy calcium carbonate filler (Escalon #800) made by Sankyo Powder Co. so that the composition in the aqueous dispersion contains 0.3 wt.% of pulp. Cationic starch (Neo-Posiparin) made by Matsutani Chemical Industry Co. of fixing agent is then added to 0.5 wt.% of pulp, and paper corresponding 80 g/m$^2$ is prepared in accordance with an ordinary process and then dried in a rotary dryer at 110°C for 120 seconds to prepare paper.

The obtained paper is measured for sizing degree after being moistened in accordance with JIS, and is listed in Table 2.

Table 2 shows the compositions which are allowed to stand for ten days, and the results of similar tests to the previous examples are listed together with emulsion mean particle size (micron) of aqueous dispersions.

TABLE 2

| Composition | Prepared composition | | Composition after 10 days | |
|---|---|---|---|---|
| | Stockigt sizing (sec.) | Mean particle size (micron) of emulsion | Stockigt sizing (sec.) | Mean particle size (micron) of emulsion |
| (a) | 38 | <1 | 36 | <1 |
| (b) | 20 | approx. 1 | 18 | approx. 1 |
| (c) | 46 | <1 | 40 | <1 |
| (d) | 23 | approx. 1 | 19 | approx. 1 |
| | | | | |
| (1) | 30 | approx. 1 | 5 | 5—6 |
| (2) | 11 | approx. 1 | 0 | 5—6 |

Example 2

Comparison Example 2

Alkenyl substituted succinic anhydride and surfactant as listed in the columns of Table 3 are both agitated and mixed under slow heating conditions at ambient temperature or at 40°C or lower, and sizing compositions (e), (f), (g), (h) of the present invention and compositions (3), (4) for comparison are prepared.

The alkenyl substituted succinic anhydride A is maleic anhydride reacted with inner olefin having 15—18 carbon atoms, and the alkenyl substituted succinic anhydride B is maleic anhydride reacted with oligomer mainly containing pentamer of propylene.

TABLE 3

| Composition | Substituted alkenyl succinic anhydride | Surfactant |
|---|---|---|
| (e) | A<br>95 wt.% | Phosphates of polyoxyethylene nonyl phenol ether ester (mono-, di-mixture)<br>(polyoxyethylene: n=9)<br>5 wt.% |
| (f) | B<br>96 wt.% | ditto<br>4 wt.% |
| (g) | A<br>95 wt.% | Phosphates of polyoxyethylene tridecyl ether ester (mono-, di-mixture)<br>(polyoxyethylene: n=9)<br>5 wt.% |
| (h) | A<br>97 wt.% | Phosphates of polyoxyethylene nonyl phenol ether ester (mono-, di-mixture)<br>(polyoxyethylene: n=21.5)<br>3 wt.% |
| (3) | A<br>95 wt.% | Polyethylene nonyl phenol ether (polyoxyethylene: n=15)<br>5 wt.% |
| (4) | A<br>96 wt.% | Sulfates of polyoxyethylene olenyl ether ester ammonium (polyoxyethylene: n=18)<br>4 wt.% |

Example 3
Comparison Example 3

Compositions (e) to (h) and (3), (4) prepared in Example 2 and Comparison Example 2 are added to 5 wt.% of aqueous solution of aqueous soluble polymer compound listed in the respective columns of Table 4 and are agitated at 50 V for 2 min in a homomixer (HV-M type made by Tokushu Kika Kogyo Co.), thereby preparing aqueous dispersions.

The prepared aqueous dispersions are added to 2 wt.% pulp slurry containing 20 wt.% of pulp (L-BKP, c.s.F. 400 cc) of heavy calcium carbonate filler (Escalon #800) made by Sankyo Powder Co.) so that the composition of alkenyl substituted succinic anhydride and surfactant in the emulsion contains 0.5 wt.% of pulp. Cationic starch (Neo-Posiparin) made by Matsutani Chemical Industry Co. as a fixing agent is then added to 0.5 wt.% of pulp, and paper corresponding to 80 g/m$^2$ is prepared in accordance with an ordinary paper-making process and is thereafter dried in a rotary dryer at 110°C for 120 seconds to prepare paper.

The thus obtained paper is measured for sizing degree after being moistened in accordance with JIS, and is listed in Table 4.

Table 4 shows the compositions which are allowed to stand for ten days, and the results of similar tests to the previous examples are listed together with emulsion mean particle size (micron) of aqueous dispersions.

TABLE 4

| Composition | | Aqueous polymer | Part of solution to part of composition (part) | Prepared composition | | Composition after 10 days | |
|---|---|---|---|---|---|---|---|
| | | | | Stockigt sizing (sec.) | Mean particle size of emulsion (micron) | Stockigt sizing (sec.) | Mean particle size of emulsion (micron) |
| (1) | (e) | Cationic Starch | 40 | 37 | 1 | 31 | 1 |
| (2) | (e) | 90 mol % of acrylamide, 10 mol % of dimethylamino-ethylmethacrylate copolymer | 10 | 24 | 1 | 22 | 1 |
| (3) | (f) | ditto to (1) | 40 | 20 | approx. 1 | 19 | approx. 1 |
| (4) | (g) | ditto to (1) | 40 | 33 | 1 | 30 | 1 |
| (5) | (h) | 95 mol % of acrylamide, 5 mol % of dimethylamino-ethylmethacrylate benzyl chloride | 10 | 28 | approx. 1 | 27 | approx. 1 |
| (6) | (3) | ditto to (1) | 40 | 30 | 1 | 19 | 3—4 |
| (7) | (4) | ditto to (1) | 40 | 32 | approx. 1 | 17 | 4—5 |

As described above, the sizing composition according to the present invention and the process for using the composition provide excellent storage stability and underwater self-emulsion, and the composition permits the production of paper having excellent sizing effect.

**Claims**

1. A sizing composition comprising (a) 70—99.9 wt.% of an alkyl- or alkenyl substituted succinic anhydride or a mixture of these succinic anhydrides, and (b) 0.1—30 wt.% of polyoxyethylene alkyl ether phosphates or polyoxyethylene alkyl aryl ether phosphates or a mixture of these phosphates.

2. A sizing composition according to claim 1, wherein the alkyl- or alkenyl substituted succinic anhydride is an addition reaction product of a straight chain inner olefin having 12 to 36 carbon atoms and maleic anhydride.

3. A sizing composition according to claim 1 or claim 2, wherein the polyoxyethylene alkyl ether phosphates or polyoxyethylene alkyl aryl ether phosphates are compounds represented by the general formula:

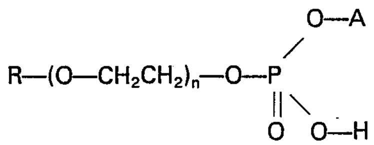

where R is an alkyl or alkyl aryl group having 8 or more carbon atoms, A represents H, or $R'—(O—CH_2CH_2)_m—$ (R' is alkyl or alkyl aryl group, m is an integer of 1 or more), and n is an integer of 1 or more.

4. A sizing composition according to claim 3, wherein R is alkyl or alkyl aryl having 10—20 carbon atoms and the value of n is 5 or more.

5. A process for using a composition as a sizing agent which comprises dispersing a sizing composition in an aqueous solution of an aqueous soluble polymer compound, adding the thus-obtained aqueous dispersion to a pulp slurry of paper making material, and making the paper, characterised in that the sizing composition is one according to any one of claims 1 to 4.

**Patentansprüche**

1. Schlichtemittel-Zusammensetzung umfassend

(a) 70 bis 99,9 Gew.-% eines Alkyl- oder Alkenyl-substituierten Bernsteinsäureanhydrids oder eine Mischung dieser Bernsteinsäureanhydride, und

(b) 0,1 bis 30 Gew.-% Polyoxyethylen-alkyletherphosphate, Polyoxyethylen-alkylaryletherphosphate oder eine Mischung dieser Phosphate.

2. Schlichtemittel-Zusammensetzung gemäß Anspruch 1, in welcher das Alkyl- oder Alkenylsubstituierte Bernsteinsäureanhydrid ein Additionsreaktionsprodukt aus einem geradkettigen inneren Olefin mit 12 bis 36 Kohlenstoffatomen und Maleinsäureanhydrid ist.

3. Schlichtemittel-Zusammensetzung gemäß Anspruch 1 oder 2, in welcher die Polyoxyethylen-alkyletherphosphate oder Polyoxyethylen-alkylaryletherphosphate Verbindungen der allgemeinen Formel

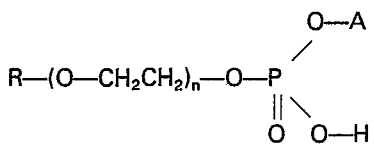

sind, worin R eine Alkyl- oder Alkylaryl-Gruppe mit 8 oder mehr Kohlenstoffatomen ist, A Wasserstoff oder $R'—(O—CH_2CH_2)_m—$ darstellt (R' ist eine Alkyl- oder Alkylaryl-Gruppe, m ist eine ganze Zahl von 1 oder mehr und n ist eine ganze Zahl von 1 oder mehr).

4. Schlichtemittel-Zusammensetzung gemäß Anspruch 3, in welcher R Alkyl- oder Alkylaryl mit 10 bis 20 Kohlenstoffatomen ist und der Wert von n 5 oder mehr ist.

5. Verfahren zur Verwendung einer Zusammensetzung als Schlichtemittel, welches das Dispergieren einer Schlichtemittel-Zusammensetzung in einer wäßrigen Lösung einer wasserlöslichen Polymerverbindung, die Zugabe der so erhaltenen wäßrigen Dispersion zu einer Pulpeaufschlämmung aus Papierherstellungsmaterial und die Papierherstellung umfaßt, dadurch gekennzeichnet, daß die Schlichtemittel-Zusammensetzung eine gemäß einem der Ansprüche 1 bis 4 ist.

**Revendications**

1. Composition de colle comprenant (a) 70 à 99,9% en poids d'un anhydride succinique à substitution

alkyle ou alcényle ou un mélange de ces anhydrides succiniques, et (b) 0,1 à 30% en poids de phosphates d'éthers alkyliques de polyoxyéthylène ou de phosphates d'éthers alkylaryliques de polyoxyéthylène ou un mélange de ces phosphates.

2. Composition de colle suivant la revendication 1, dans laquelle l'anhydride succinique à substitution alkyle ou alcényle est un produit de réaction d'addition d'une oléfine interne à chaîne droite ayant 12 à 36 atomes de carbone et de l'anhydride maléique.

3. Composition de colle suivant la revendication 1 ou la revendication 2, dans laquelle les phosphates d'éthers alkyliques de polyoxyéthylène ou les phosphates d'éthers alkylaryliques de polyoxyéthylène sont des composés représentés par la formule générale

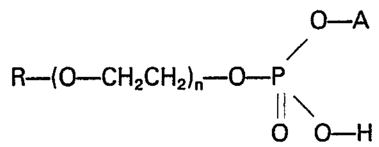

dans laquelle R est un groupe alkyle ou alkylaryle ayant un nombre d'atomes de carbone égal ou supérieur à 8, A représente H ou $R'—(O—CH_2CH_2)_m—$ (R' est un groupe alkyle ou alkylaryle, m est un nombre entier égal ou supérieur à 1), et n est un nombre entier égal ou supérieur à 1.

4. Composition de colle suivant la revendication 3, dans laquelle R est un groupe alkyle ou alkylaryle ayant 10 à 20 atomes de carbone et n a une valeur égale ou supérieure à 5.

5. Procédé d'utilisation d'une composition comme agent de collage, qui consiste à disperser une composition de colle dans une solution aqueuse d'un composé polymérique hydrosoluble, à ajouter la dispersion aqueuse ainsi obtenue à une suspension de pâte de matière destinée à la production du papier, et à produire le papier, caractérisé en ce que la composition de colle est une composition suivant l'une quelconque des revendications 1 à 4.